# EUROPÄISCHE PATENTSCHRIFT

(11) **EP 0 907 736 B1**
(45) Veröffentlichungstag und Bekanntmachung des Hinweises auf die Patenterteilung: **01.02.2006**
(21) Anmeldenummer: 97921835.1
(22) Anmeldetag: 30.04.1997
(51) Int. Cl.: C12N 15/19, C07K 14/52, A61K 38/19, G01N 33/50, C07H 21/00, C12Q 1/68

(54) **CHEMOKINE VOM CC-TYP**
CC-TYPE CHEMOKINES
CHEMOKINES DE TYPE CC

(30) Priorität: 30.04.1996 DE 19617312
(43) Veröffentlichungstag der Anmeldung: 14.04.1999
(73) Patentinhaber: Pharis Biotec GmbH, 30625 Hannover (DE)
(72) Erfinder: FORSSMANN, Wolf-Georg, N.sächs.Inst.Peptid-F.(IPF), D-30625 Hannover (DE); PARDIGOL, Andreas, Niedersächs.Inst.Peptid-F.(IPF), D-30625 Hannover (DE); MÄGERT, Hans-Jürgen, N.sächs.Inst.Peptid-F.(IPF), D-30625 Hannover (DE); SCHULZ-KNAPPE, Peter, N.sächs.Inst.Peptid-F.(IPF), D-30625 Hannover (DE)
(74) Vertreter: Meyers, Hans-Wilhelm
(86) Internationale Anmeldenummer: PCT/EP1997/002217
(87) Internationale Veröffentlichungsnummer: WO 1997/041230

(56) Entgegenhaltungen:
- WO-A-95/17092
- WO-A-95/18228
- WO-A-96/16979
- WO-A-96/34891
- WO-A-97/21812
- DE-A- 19 512 463
- SCHULZ-KNAPPE P ET AL: "HCC-1, a novel chemokine from human plasma" JOURNAL OF EXPERIMENTAL MEDICINE, 183 (1). JAN 1996. 295-299., XP002044126

## Beschreibung

Gegenstand der vorliegenden Erfindung ist die Nukleinsäure eines Tandem-Gens, das zwei Polypeptide aus der Klasse der Cytokine kodiert, die Cytokine CC-2 und CC-3 sowie deren biologisch aktive Fragmente und/oder Derivate, ein Arzneimittel enthal end die erfindungsgemäßen Peptide, ein Diagnostikmittel, sowie die Verwendung von Cytokin CC-2 und CC-3 für medizinische Indikationen.

Die Chemokine vom CC-Typ gehören zu einer Familie von Polypeptiden, die sich als Vermittler von Immunreaktionen erwiesen haben, und erregen neuerdings Aufmerksamkeit wegen ihrer antiviralen Wirkung in bezug auf HIV.

Die Chemokine sind kleine basische, heparinbindende Polypeptide, die an der Erzeugung von Immunreaktionen und an Entzündungsvorgängen beteiligt sind (1, 2). Die ersten Mitglieder dieser Familie wurden in der Mitte der 80er Jahre aus stimulierten tonsillären Lymphozyten oder Makrophagen kloniert (3, 4). Die Familie wird nach der relativen Lage der ersten beiden Cysteinreste in CXC- und CC-Chemokine unterteilt. Die meisten CXC- und CC-Chemokine sind chemotaktisch in bezug auf Zellen, die an Immunreaktionen beteiligt sind. Einige CC-Chemokine zeigen multiple Bioaktivitäten, wie Steuerung der Vermehrung und Differenzierung hämatopoetischer Vorläuferzellen (5, 6, 7, 8, 9), Aktivierung und Attraktion eines breiten Spektrums von Immunzellen (10, 11) und die postulierte Beteiligung bei der Steuerung der Replikation von HIV-1 und HIV-2 in CD-4-positiven T-Lymphozyten (12). Zu den am meisten beschriebenen und am besten untersuchten CC-Chemokinen im oben beschriebenen Zusammenhang gehören MIP-1α, MIP-1β, und RANTES. Die Unterteilung der Chemokine in CC- und CXC-Chemokine spiegelt sich auch in der Struktur und der Position ihrer Gene auf den Chromosomen wider (14, 15, 16, 17). Im allgemeinen befinden sich Gene für Human-CXC-Chemokine auf dem Chromosom 4 und bestehen aus vier Exons und drei Introns, während die Gene für CC-Chemokine auf dem Chromosom 17 des Menschen liegen und eine konservierte Struktur mit drei Exons und zwei Introns zeigen (18, 19). Im Jahre 1995 wurde ein neuer Typ von Chemokinen entdeckt, die sogenannte C-Familie, die durch Maus-Lymphotaktin vertreten ist (20). Dieser Befund zeigt, daß die Chemokine nicht auf nur zwei Unterfamilien beschränkt sind.

Die Erfindung betrifft unter anderem die Klonierung eines Human-Tandem-Gens sowie die entsprechende reife bicistronische mRNA, die die offenen Leseraster (ORF) für ein neues CC-Chemokin mit sechs Cysteinresten, das CC-2, sowie für den zuvor beschriebenen Faktor HCC-1 enthält. Dieser Faktor wurde aus Human-Hämofiltrat isoliert, und es wurde gezeigt, daß er in hohen Konzentrationen im Human-Plasma gesunder Individuen (1-5 nM) sowie von Patienten mit Nierenversagen (5-80 nM) zirkuliert (21). Im Unterschied zu anderen, verwandten CC-Chemokinen zeigt HCC-1 keine Chemoaktivität gegenüber Monocyten und stimuliert nur leicht die intrazelluläre Ca²⁺-Mobilisierung sowie die Enzymfreisetzung in diesen Zellen. Weiterhin wurde berichtet, daß HCC-1 die Vermehrung CD-34-positiver Knochenmark-Stammzellen in dosisabhängiger Weise fördert (21). Trotz der beschriebenen Wirkungen von HCC-1 bleibt seine hauptsächliche biologische Funktion unklar.

Die erfindungsgemäße Nukleinsäure eines humanen Tandem-Gens kodiert für zwei neue Chemokine vom CC-Typ, deren Sequenzen irrtümlicherweise mit der von MIP-1α in gewissem Maß homolog sind.

Gegenstand der vorliegenden Erfindung ist somit die Nukleinsäure des Tandem-Gens mit den folgenden Sequenzen: 
Sequence 925 BP; 240 A; 296 C; 199 G; 190 T;
CCAGGAAGCA GTGAGCCCAG GAGTCCTCGG CCAGCCCTGC CTGCCCACCA GGAGGATGAA
GGTCTCCGTG GCTGCCCTCT CCTGCCTCAT GCTTGTTGCT GTCCTTGGAT CCCAGGCCCA
GTTCACAAAT GATGCAGAGA CAGAGTTAAT GATGTCAAAG CTTCCACTGG AAAATCCAGT
AGTTCTGAAC AGCTTTCACT TTGCTGCTGA CTGCTGCACC TCCTACATCT CACAAAGCAT
CCCGTGTTCA CTCATGAAAA GTTATTTTGA AACGAGCAGC GAGTGCTCCA AGCCAGGTGT
CATATTCCTC ACCAAGAAGG GGCGGCAAGT CTGTGCCAAA CCCAGTGGTC CGGGAGTTCA
GGATTGCATG AAAAAGCTGA AGCCCTACTC AATATAATAA TAAAGAGACA AAAGAGGCCA
GCCACCCACC TCCAACACCT CCTGAGCCTC TGAAGCTCCC ACCAGGCCAG CTCTCCTCCC
ACAACAGCTT CCCACAGCAT GAAGATCTCC GTGGCTGCCA TTCCCTTCTT CCTCCTCATC
ACCATCGCCC TAGGGACCAA GACTGAATCC TCCTCACGGG GACCTTACCA CCCCTCAGAG
TGCTGCTTCA CCTACACTAC CTACAAGATC CCGCGTCAGC GGATTATGGA TTACTATGAG
ACCAACAGCC AGTGCTCCAA GCCCGGAATT GTCTTCATCA CCAAAAGGGG CCATTCCGTC
TGTACCAACC CCAGTGACAA GTGGGTCCAG GACTATATCA AGGACATGAA GGAGAACTGA
GTGACCCAGA AGGGGTGGCG AAGGCACAGC TCAGAGACAT AAAGAGAAGA TGCCAAGGCC
CCCTCCTCCA CCCACCGCTA ACTCTCAGCC CCAGTCACCC TCTTGGAGCT TCCCTGCTTT
GAATTAAAGA CCACTCATGC TCTTC
und 
Sequence 973 BP; 257 A; 301 C; 215 G; 200 T;
CCAGGAAGCA GTGAGCCCAG GAGTCCTCGG CCAGCCCTGC CTGCCCACCA GGAGGATGAA
GGTCTCCGTG GCTGCCCTCT CCTGCCTCAT GCTTGTTGCT GTCCTTGGAT CCCAGGCCCA
GTTCACAAAT GATGCAGAGA CAGAGTTAAT GATGTCAAAG CTTCCACTGG AAAATCCAGT
AGTTCTGAAC AGCTTTCACT TTGCTGCTGA CTGCTGCACC TCCTACATCT CACAAAGCAT
CCCGTGTTCA CTCATGAAAA GTTATTTTGA AACGAGCAGC GAGTGCTCCA AGCCAGGTGT
CATATTCCTC ACCAAGAAGG GGCGGCAAGT CTGTGCCAAA CCCAGTGGTC CGGGAGTTCA
GGATTGCATG AAAAAGCTGA AGCCCTACTC AATATAATAA TAAAGAGACA AAAGAGGCCA
GCCACCCACC TCCAACACCT CCTGAGCCTC TGAAGCTCCC ACCAGGCCAG CTCTCCTCCC
ACAACAGCTT CCCACAGCAT GAAGATCTCC GTGGCTGCCA TTCCCTTCTT CCTCCTCATC
ACCATCGCCC TAGGGACCAA GACTGAATCC TCCTCACAAA CTGGGGGGAA ACCGAAGGTT
GTTAAAATAC AGCTAAAGTT GGTGGGGGGA CCTTACCACC CCTCAGAGTG CTGCTTCACC
TACACTACCT ACAAGATCCC GCGTCAGCGG ATTATGGATT ACTATGAGAC CAACAGCCAG
TGCTCCAAGC CCGGAATTGT CTTCATCACC AAAAGGGGCC ATTCCGTCTG TACCAACCCC
AGTGACAAGT GGGTCCAGGA CTATATCAAG GACATGAAGG AGAACTGAGT GACCCAGAAG
GGGTGGCGAA GGCACAGCTC AGAGACATAA AGAGAAGATG CCAAGGCCCC CTCCTCCACC
CACCGCTAAC TCTCAGCCCC AGTCACCCTC TTGGAGCTTC CCTGCTTTGA ATTAAAGACC
ACTCATGCTC TTC
kodierend für die humanen Cytokine CC-2 und CC-3 mit den nachfolgenden Aminosäuresequenzen

CC-2:
M K
ATG AAG

V S V A A L S C L M L V A V L G S Q A Q
GTC TCC GTG GCT GCC CTC TCC TGC CTC ATG CTT GTT GCT GTC CTT GGA TCC CAG GCC CAG

F T N D A E T E L M M S K L P L E N P V
TTC ACA AAT GAT GCA GAG ACA GAG TTA ATG ATG TCA AAG CTT CCA CTG GAA AAT CCA GTA

V L N S F H F A A D C C T S Y I S Q S I
GTT CTG AAC AGC TTT CAC TTT GCT GCT GAC TGC TGC ACC TCC TAC ATC TCA CAA AGC ATC

P C S L M K S Y F E T S S E C S K P G V
CCG TGT TCA CTC ATG AAA AGT TAT TTT GAA ACG AGC AGC GAG TGC TCC AAG CCA GGT GTC

I F L T K K G R Q V C A K P S G P G V Q
ATA TTC CTC ACC AAG AAG GGG CGG CAA GTC TGT GCC AAA CCC AGT GGT CCG GGA GTT CAG

D C M K K L K P Y S I TER TER TER
GAT TGC ATG AAA AAG CTG AAG CCC TAC TCA ATA TAA TAA TAAAGAGACAAAAGAGGCCAGCCACCC

M K
ACCTCCAACACCTCCTGAGCCTCTGAAGCTCCCACCAGGCCAGCTCT[?]C[?]CCCACAACAGCTTCCCACAGC ATG AAG

I S V A A I P F F L L I T I A L G T K T
ATC TCC GTG GCT GCC ATT CCC TTC TTC CTC CTC ATC ACC ATC GCC CTA GGG ACC AAG ACT

E S S S R G P Y H P S E C C F T Y T T Y
GAA TCC TCC TCA CGG GGA CCT TAC CAC CCC TCA GAG TGC TGC TTC ACC TAC ACT ACC TAC

K I P R Q R I M D Y Y E T N S Q C S K P
AAG ATC CCG CGT CAG CGG ATT ATG GAT TAC TAT GAG ACC AAC AGC CAG TGC TCC AAG CCC

G I V F I T K R G H S V C T N P S D K W
GGA ATT GTC TTC ATC ACC AAA AGG GGC CAT TCC GTC TGT ACC AAC CCC AGT GAC AAG TGG

V Q D Y I K D M K E N
GTC CAG GAC TAT ATC AAG GAC ATG AAG GAG AAC

CC-3:
M K
ATG AAG
V S V A A L S C L M L V A V L G S Q A Q
GTC TCC GTG GCT GCC CTC TCC TGC CTC ATG CTT GTT GCT GTC CTT GGA TCC CAG GCC CAG
F T N D A E T E L M M S K L P L E N P V
TTC ACA AAT GAT GCA GAG ACA GAG TTA ATG ATG TCA AAG CTT CCA CTG GAA AAT CCA GTA
V L N S F H F A A D C C T S Y I S Q S I
GTT CTG AAC AGC TTT CAC TTT GCT GCT GAC TGC TGC ACC TCC TAC ATC TCA CAA AGC ATC
P C S L M K S Y F E T S S E C S K P G V
CCG TGT TCA CTC ATG AAA AGT TAT TTT GAA ACG AGC AGC GAG TGC TCC AAG CCA GGT GTC
I F L T K K G R Q V C A K P S G P G V Q
ATA TTC CTC ACC AAG AAG GGG CGG CAA GTC TGT GCC AAA CCC AGT GGT CCG GGA GTT CAG
D C M K K L K P Y S I TER TER TER
GAT TGC ATG AAA AAG CTG AAG CCC TAC TCA ATA TAA TAA TAAAGAGACAAAAGAGGCCAGCCACCC
M K
ACCTCCAACACCTCCTGAGCCTCTGAAGCTCCCACCAGGCCAGCTCTCCTCCCACAACAGCTTCCCACAGC ATG AAG
I S V A A I P F F L L I T I A L G T K T
ATC TCC GTG GCT GCC ATT CCC TTC TTC CTC CTC ATC ACC ATC GCC CTA GGG ACC AAG ACT
E S S S Q T G G K P K V V K I Q L K L V
GAA TCC TCC TCA CAA ACT GGG GGG AAA CCG AAG GTT GTT AAA ATA CAG CTA AAG TTG GTG
G G P Y H P S E C C F T Y T T Y K I P R
GGG GGA CCT TAC CAC CCC TCA GAG TGC TGC TTC ACC TAC ACT ACC TAC AAG ATC CCG CGT
Q R I M D Y Y E T N S Q C S K P G I V F
CAG CGG ATT ATG GAT TAC TAT GAG ACC AAC AGC CAG TGC TCC AAG CCC GGA ATT GTC TTC
I T K R G H S V C T N P S D K W V Q D Y
ATC ACC AAA AGG GGC CAT TCC GTC TGT ACC AAC CCC AGT GAC AAG TGG GTC CAG GAC TAT
I K D M K E N
ATC AAG GAC ATG AAG GAG AAC

Die Transkription des Tandem-Gens führt zu einem bicistronischen reifen Transkript, das die nichtüberlappenden offenen Leseraster für den kürzlich beschriebenen Faktor HCC-1 und CC-2 enthält. Durch alternatives Spleißen des primären Transkripts entsteht überdies wenigstens ein weiteres Chemokin vom CC-Typ, das Cytokin CC-3. Diese neuen Cytokine vom CC-Typ sind ebenfalls Gegenstand der Erfindung sowie deren biologisch aktive amidierte, acetylierte, phosphorylierte und/oder glycosylierte Derivate.

Innerhalb des Tandem-Gens konnten zwei funktionelle Promotorbereiche identifiziert werden. Es wurde deren Fähigkeit untersucht, die Expression des Tandem-Gens zu induzieren.

Die ermittelten Daten liefern ein Grundwissen über die Struktur und die Expression des neuen humanen CC-2/HCC-1-Tandem-Gens und beschreiben einen Mechanismus, nach dem die Coexpression eng miteinander verknüpfter Gene bei höheren Eukaryonten reguliert werden könnte.

Erfindungsgemäß beansprucht wird ein Arzneimittel enthaltend Cytokin CC-2 und/oder CC-3 sowie deren biologisch aktive amidierte, acetylierte, phosphorylierte und/oder glycosylierte Derivate als wirksamen Bestandteil. Das Arzneimittel wird in entsprechender, an seine Applikationsart angepaßter und geeigneter Galenik verabreicht. Das erfindungsgemäße Arzneimittel kann dabei in für Peptide üblicher Weise parenteral, intravenös oder intramuskulär oder intranasal, bukkal verabreicht werden. Die Menge an zu verabreichenden Peptid beträgt zwischen 10 und 3.000 µg pro Darreichungseinheit.

Das erfindungsgemäße Diagnostikmittel enthält poly- oder monoklonale Antikörper gegen das erfindungsgemäße Peptid gegebenenfalls in fluoreszenz- oder radioaktivmarkierter Form um in an sich bekannten ELISA oder RIA Assays eingesetzt zu werden.

Die erfindungsgemäßen Peptide Cytokin CC-2 und/oder CC-3 können zur Herstellung eines Arzneimittels zur Behandlung von Störungen der Migration von Zellen, Erkrankungen des Immunsystems, Tumoren sowie Disfunktion regulatorischer Wachstumsfunktionen verwendet werden.

Die cDNA der Sequenz der bicistronischen CC-2/HCC-1-cDNA mit der vollen Länge wurde durch 5'-RACE-PCR unter Verwendung von 5 µg Gesamt-RNA aus T-84-Zellen erhalten. Das PCR-Fragment wurde direkt aus dem ersten Strang adulter Leber-cDNA sequenziert und reamplifiziert, wobei ein Oligo-dT-Primer in Verbindung mit einem spezifischen Primer verwendet wurde (35 Cyclen); danach folgte ein zweiter Amplifikationsschritt (30 Cyclen) mit eingenisteten Primern. Eine computergestützte Sequenzanalyse bestätigte zwei offene Leseraster (ORF) innerhalb der cDNA. Das stromaufwärts gelegene ORF codiert für eine hypothetische Vorstufe mit einer Länge von 113 Aminosäuren (aa). Interessanterweise wird die Translation des ersten ORF durch drei Stopcodons beendet. Ein Hydrophobie-Plot ergab eine N-terminale hydrophobe Sequenz von 20 aa, bei der es sich um ein Leitpeptid handeln könnte. Die Sequenz des hypothetischen Peptids, das wir CC-2 nannten, ist zu 46% zu der von MIP-1α homolog, enthält jedoch zwei zusätzliche Cysteinreste. Das stromabwärts gelegene ORF codiert für HCC-1. Alternatives Spleißen innerhalb des Introns 1 des primären Transcripts führt zu einer Peptidvariation von HCC-1, die 16 zusätzliche Aminosäuren enthält.

Die Struktur des Human-CC-2/HCC-1-Tandem-Gens wurde bestimmt. Das 20-kbp-Tandem-Gen, das aus einem λ-FIX-II-Phagen isoliert wurde, welcher aus einer Human-Genbank isoliert wurde, enthält sieben Exons, von denen die Exons α-δ das hypothetische Peptid CC-2 codieren und das die Exons 1-3 HCC-1 codieren. Der gesamte Bereich wurde durch Restriktionsanalyse kartiert. Die Reihenfolge und Orientierung der SstI-Restriktionsfragmente wurde durch Sequenzieren der flankierenden Bereiche jeder SstI-Restriktionsstelle ermittelt. Die Sequenzierung wurde mit Hilfe eines automatischen Fluoreszenz-Sequenzers durchgeführt. Restriktionsfragmente, die das HCC-1-Gen enthielten, wurden durch Southern-Blot-Analyse mit der HCC-1-cDNA als radioaktiver Sonde nachgewiesen. Die fünfzehn terminalen bp der monocistronischen HCC-1-cDNA treten in der bicistronischen mRNA nicht auf; daher sind sie gleichzeitig ein Teil von Exon 1 und Intron δ. Die CC-2-Gensequenz wurde durch Primer-Walking bestimmt. Alle Exon/Intron-Spleißverbindungen stimmen gut mit den Consensussequenzen überein: Die Primersequenzen wurden aus der bicistronischen CC-2-cDNA abgeleitet. Die Aktivität beider Promotoren wurde durch einen Luciferase-Reporter-Gen-Assay untersucht. Die Transcriptionsstartpunkte wurden zuvor durch Primerverlängerung, halbquantitative RT-PCR und 5'-RACE bestimmt.

Die Erfindung wird anhand der folgenden Beispiele näher erläutert.

Um die Regulation des HCC-1-Gens zu untersuchen, wurde das für HCC-1 codierende Gen unter Verwendung einer partiellen HCC-1-cDNA als Hybridisierungssonde kloniert. Die Sequenz analyse zeigte, daß die Organisation des HCC-1 gut mit der beschriebenen Struktur aus drei Exons und zwei Introns im Einklang steht, die für CC-Chemokin-Gene typisch ist. Um eine Chromosom-Kartierung des Gens zu erhalten, wurde eine PCR durchgeführt, wobei DNA von drei Mäusezellen-Hybriden verwendet wurde, die jeweils eine definierte Menge menschlicher Chromosomen enthielten. Die Ergebnisse weisen darauf hin, daß das HCC-1-Gen auf Chromosom 17 liegt und daher der Unterfamilie der CC-Chemokin-Gene zugeordnet werden kann.

Zur Untersuchung der Aktivität des HCC-1-Promotors wurde wie folgt vorgegangen. Zunächst wurde der Transkriptionsstartpunkt mittels Primer-Verlängerung und halbquantitativer RT-PCR-Analyse (PCR mit Revers Transcriptase) bestimmt, was zeigte, daß in der Leber eine alternative Transcriptionsinitiation vorlag, im Gegensatz zu Knochenmark und Mandeln. Etwa 700 bp des stromaufwärts gelegenen Bereichs, die vermutliche regulatorische Elemente sowie ein TATA-Box-Motiv enthielten, wurden vor dem Luciferase-Reporter-Gen in den pGL-2-Grundvektor einkloniert. Die Assays für das Reporter-Gen wurden durchgeführt, indem man drei verschiedene Zellinien, die als HUH-7, T 84 und RK 13 bezeichnet werden, mit dem genannten HCC-1-Promotor/-Reporter-Gen-Konstrukt sowie fünf am 5'-Ende verkürzten Deletionskonstrukten transfizierte. Die HUH-7- und T-84-Zellen wurden vor der Transfektion auf Expression des HCC-1-Gens hin getestet, und beide Zellinien exprimierten offenbar das HCC-1 -Gen. Überraschenderweise konnte in HUH-7- und T-84-Zellen nur eine schwache Promotoraktivität (das 2- bis 3 fache der Hintergrundaktivität) gemessen werden, und eine geringe Aktivität (das 6fache der Hintergrundaktivität) wurde in RK-13-Zellen nachgewiesen. Eine Northern-Blot-Analyse zeigte jedoch starke HCC-1-mRNA-Signale in Leber und deutlich nachweisbare Signale im Dickdarm (21), was vermuten läßt, daß, die Aktivität des HCC-1-Promotors in vivo von einem Enhancer-Element abhängt, das in den Reporter-Gen-Konstrukten fehlte.

Während einer Routinesequenzrecherche in einer Datenbank mit Markierungen für exprimierte Sequenzen (EST) wurde eine 5'-verlängerte partielle HCC-1-cDNA-Sequenz gefunden. Die zusätzliche 5'-terminale Sequenz war nicht in dem regulatorischen HCC-1-Upstream-Bereich vertreten, der auf Promotoraktivität hin getestet worden war, was daher die Existenz wenigstens eines weiteren, stromaufwärts gelegenen, nicht translatierten Exons vermuten ließ. Interessanterweise waren die fünfzehn 5'-terminalen Nucleotide der HCC-1-cDNA (21) in der verlängerten cDNA nicht vertreten, was beweist, daß das Fragment, das in dem Reporter-Gen-Assay getestet worden war, ein Teil des HCC-1-Promotors ist. Diese Befunde deuteten überraschenderweise darauf hin, daß es einen zweiten Promotorbereich vor dem ersten nicht translatierten Exon gab. Um den zweiten Promotor innerhalb des Gens zu lokalisieren, wurde zunächst die vollständige verlängerte HCC-1-cDNA mit Hilfe des 5'-RACE-PCR-Verfahrens isoliert. cDNA des ersten Stranges wurde dabei verwendet, die aus der Gesamt-RNA von T-84-Zellen synthetisiert wurde. Das erhaltene Produkt enthielt gegenüber der veröffentlichten HCC-1-cDNA-Sequenz 441 zusätzliche Nucleotide. Es ist bemerkenswert, das in der Datenbank ein EST auffindbar war, das zum 5'-Terminus des RACE-PCR-Produkts paßt. Dies erhöht die Wahrscheinlichkeit, daß die verlängerte cDNA der vollen Länge entspricht. Eine Analyse der cDNA-Sequenz zeigte, daß das erste 5'-terminale ORF der cDNA kein HCC-1 codiert, sondern für ein neues CC-Chemokin codiert, das CC-2. Das aus dieser cDNA-Sequenz abgeleitete Propeptid besteht aus 113 Aminosäuren und enthält eine hydrophobe N-terminale Sequenz aus 20 Aminosäuren mit typischen Merkmalen eines Leitpeptids. Eine Sequenzvergleichsrecherche zeigte, daß die Precursor von CC-2 und MIP-1α eine zu 46% identische Sequenz haben. Das hypothetische CC-2 enthält jedoch sechs Cysteinreste, was vor kurzem auch für zwei neue Maus-CC-Chemokine, C 10 (22) und CCF 18 (23), beschrieben wurde, die nur eine geringe Sequenzhomologie zu CC-2 zeigen, aber über dasselbe Cysteinmuster verfügen. Das Human-CC-Chemokin I-309 (27) enthält ebenfalls sechs Cysteinreste, weist aber nicht die gleiche relative Position bei den zwei zusätzlichen Cysteinresten auf. Daher könnten CC-2, C 10 und CCF 18 eine neue Untergruppe von CC-Chemokinen bilden, die bei Menschen und Mäusen konserviert sind und eine zusätzliche hypothetische Disulfidbindung tragen. Um nachzuweisen, daß das bicistronische Transkript auch in nichttransformierten Gewebezellen synthetisiert wird, wurde die cDNA der vollen Länge aus dem ersten Strang der cDNA aus adulter Leber isoliert und sequenziert. Die offenen Leseraster für CC-2 und HCC-1 überlappen nicht und liegen 101 Nucleotide weit auseinander. Ein sequenzierter cDNA-Klon, enthielt 48 zusätzliche Nucleotide im stromabwärts gelegenen codierenden Bereich, was zu einer Peptidvariation von HCC-1 führt, die 16 zusätzliche Aminosäuren enthält. Dieses CC-Chemokin wurde CC-3 genannt. Ein Vergleich mit der genomischen Sequenz des HCC-1-Gens zeigte, daß das zusätzliche Exon innerhalb des Introns 1 liegt und durch alternatives Spleißen des primären Transcripts entsteht.

Weiterhin wurde die Struktur der genomischen Sequenz von CC-2 bestimmt und die Regulation des entsprechenden Gens untersucht. Daher wurde der gesamte Einschub des λ-FIX-II-Phagen kartiert, der ursprünglich während der Durchmusterung nach dem HCC-1-Gen isoliert wurde. Es wurde ein 16-kbp-Restriktionsfragment gefunden, das den 5'-Upstream-Bereich des HCC-1-Gens enthält. Eine Sequenzanalyse bestätigte vollkommen den codierenden Bereich der CC-2-Nucleotidsequenz innerhalb dieses Fragments. Im Unterschied zu allen bekannten CC-Chemokin-Genen, die drei Exons enthalten, die durch zwei Introns voneinander getrennt sind, besteht der codierende Bereich des CC-2-Gens aus vier Exons, die durch drei Introns voneinander getrennt sind. Der Abstand zwischen dem Ende von Exon δ des CC-2-Gens und Exon 1 des HCC-1-Gens beträgt ca. 12 kbp. Der Upstream-Bereich des Exons α des Tandem-Gens enthält ein TATA-Box-Motiv (TATAAAT) auf Position -32 in bezug auf den Transkriptionsstartpunkt, aber weder GC- noch CCAAT-Box-Motive. Die relative Aktivität von ca. 900 bp des vermuteten Promotors, der in den pGL-2-Grundvektor einkloniert wurde, wurde durch Transfizieren von T-84-Zellen getestet. Es wurde gezeigt, daß der Promotor eine Aktivität vom 8- bis 9fachen des Hintergrundniveaus zeigte, was beweist, daß das Tandem-Gen aktiv transcribiert wird. Zieht man die relativ schwache Aktivität der beiden Promotoren in Betracht, so ist man versucht, Vermutungen darüber anzustellen, ob die beiden Promotorbereiche positiv miteinander wechselwirken, was zu der starken konstitutiven Expression des HCC-1-Gens in der Leber und verschiedenen anderen Geweben führt. Die Existenz von zwei Promotorbereichen innerhalb des Tandem-Gen-Komplexes läßt jedoch vermuten, daß beide Transkriptionseinheiten des Tandem-Gens auch unabhängig voneinander aktiviert werden können. Dies unterstreicht die Bedeutung intergenischer Spacerbereiche, die anscheinend als Introns fungieren können und daher unter bestimmten Umständen vielleicht wichtige Bereiche innerhalb des Genoms von Eukaryonten zur Verbindung eng miteinander verknüpfter Gene: darstellen. Die Existenz bicistronischer reifer mRNAs bei Menschen und Mäusen wurde zuerst für GDF-1 und einen zweiten, unbekannten Faktor, der als UOG-1-bezeichnet wurde, beschrieben (28). Das CC-2/HCC-1-Tandem-Gen stellt daher das zweite Beispiel für eine reife bicistronische Human-mRNA dar, was die Hypothese unterstützt, daß polycistronische reife Transknipte bei höheren Eukaryonten wahrscheinlich weiter verbreitet sind. Wie kürzlich für den Transcriptionsfaktor GATA-1 (24) und einige andere Faktoren (25, 26) gezeigt wurde, ist die Verwendung interner ATG-Startcodons nicht auf prokaryontische mRNAs beschränkt, sondern tritt auch bei eukaryontischen Zellen auf. Es bleibt noch zu beweisen, ob die Translation eukaryontischer reifer polycistronischer mRNAs zu multiplen Genprodukten führt. Im Hinblick auf diesen Aspekt liefert unsere Arbeit neues Grundwissen über die Struktur und die Regulation eng miteinander verknüpfter Gene und die Bildung polycistronischer mRNAs.
1. Baggiolini, M. & Dahinden, C. A. *Immunology Today* **15***,* 127-133 (1994)
2. Furie, M. B. & Randolph, G. J. *Am. J. Pathol.* **146,** 1287-1301 (1995)
3. Obaru, K. *et al., J. Biochem* **99,** 885-894 (1986)
4. Davatelis, G. *et al., J. Exp. Med.* **167**, 1939-1944 (1988)
5. Graham, G. J. & Pragnell, I. B. *Dev. Biol.* **151**, 377-381 (1992)
6. Verfaillie, C. M. *et al., J. Exp. Med.* **179***,* 643-649 (1994)
7. Broxmeyer, H. E. *et al., J. Jmmunol.* **147**, 2586-2594 (1991)
8. Broxmeyer, H. E. *et al., J. Immunol* **150**, 3448-3458 (1993)
9. Graham, G. J. *et al., Nature* **344**, 442-444 (1990)
10. Uguccioni, M. *et al., Eur. J. Immunol.* **25**, 64-68 (1995)
11. Rot, *A. et al., J. Exp. Med.* **176**, 1489-1495 (1992)
12. Cocchi, F. *et al., Science* **270**, 1811-1815 (1995)
14. Widmer, U. *et al., J. Immunol.* **146**, 4031-4040 (1991)
15. Widmer, U. *et al., J. Immunol.* **150**, 4996-5012 (1993)
16. Napolitano, *M. et al., J. Biol. Chem.* **266**, 17531-17536 (1991)
17. Irving, S. G. *et al., Nucleic Acids Research* **18**, 3261-3270 (1990)
18. Baggiolini, M. *et al., Adv. Immunol.* **55**, 97-179 (1994)
19. Oppenheim, J. J. *et al., Annu. Rev. Immunol.* **9,** 617-648 (1991)
20. Kelner, G. S. *et al., Science* **266,** 1395-1399 (1994)
21. Schulz-Knappe, P. *et al., J. Exp. Med.* **183,** 295-299 (1996)
22. Orlofsky, A. *et al., Cell Regul.* **2**, 403-xyz (1991)
23. Hara, T. *et al., J. Immunol.* **155**, 5352-5358 (1995)
24. Calligaris, *R. et al., Proc. Natl. Acad. Sci.* **92,** 1 1598-11602 (1995)
25. Delmas, V. *et al., Proc. Natl. Acad. Sci.* **89,** 4226-4230 (1992)
26. Schöler, H. R. *et al., Nature* **344,** 435-439 (1990)
27. Miller, M. D. et al., *J. Immunol.* **143**, 2907-2916 (1989)
28. Lee, S. J*., Proc. Natl. Acad. Sci.* **88**, 4250-4254 (1991)

## Patentansprüche

1. Nukleinsäure eines Tandem-Gens mit den folgenden Sequenzen 
Sequence 925 BP; 240 A; 296 C; 199 G; 190 T;
CCAGGAAGCA GTGAGCCCAG GAGTCCTCGG CCAGCCCTGC CTGCCCACCA GGAGGATGAA
GGTCTCCGTG GCTGCCCTCT CCTGCCTCAT GCTTGTTGCT GTCCTTGGAT CCCAGGCCCA
GTTCACAAAT GATGCAGAGA CAGAGTTAAT GATGTCAAAG CTTCCACTGG AAAATCCAGT
AGTTCTGAAC AGCTTTCACT TTGCTGCTGA CTGCTGCACC TCCTACATCT CACAAAGCAT
CCCGTGTTCA CTCATGAAAA GTTATTTTGA AACGAGCAGC GAGTGCTCCA AGCCAGGTGT
CATATTCCTC ACCAAGAAGG GGCGGCAAGT CTGTGCCAAA CCCAGTGGTC CGGGAGTTCA
GGATTGCATG AAAAAGCTGA AGCCCTACTC AATATAATAA TAAAGAGACA AAAGAGGCCA
GCCACCCACC TCCAACACCT CCTGAGCCTC TGAAGCTCCC ACCAGGCCAG CTCTCCTCCC
ACAACAGCTT CCCACAGCAT GAAGATCTCC GTGGCTGCCA TTCCCTTCTT CCTCCTCATC
ACCATCGCCC TAGGGACCAA GACTGAATCC TCCTCACGGG GACCTTACCA CCCCTCAGAG
TGCTGCTTCA CCTACACTAC CTACAAGATC CCGCGTCAGC GGATTATGGA TTACTATGAG
ACCAACAGCC AGTGCTCCAA GCCCGGAATT GTCTTCATCA CCAAAAGGGG CCATTCCGTC
TGTACCAACC CCAGTGACAA GTGGGTCCAG GACTATATCA AGGACATGAA GGAGAACTGA
GTGACCCAGA AGGGGTGGCG AAGGCACAGC TCAGAGACAT AAAGAGAAGA TGCCAAGGCC
CCCTCCTCCA CCCACCGCTA ACTCTCAGCC CCAGTCACCC TCTTGGAGCT TCCCTGCTTT
GAATTAAAGA CCACTCATGC TCTTC
und 
Sequence 973 BP; 257 A; 301 C; 215 G; 200 T;
CCAGGAAGCA GTGAGCCCAG GAGTCCTCGG CCAGCCCTGC CTGCCCACCA GGAGGATGAA
GGTCTCCGTG GCTGCCCTCT CCTGCCTCAT GCTTGTTGCT GTCCTTGGAT CCCAGGCCCA
GTTCACAAAT GATGCAGAGA CAGAGTTAAT GATGTCAAAG CTTCCACTGG AAAATCCAGT
AGTTCTGAAC AGCTTTCACT TTGCTGCTGA CTGCTGCACC TCCTACATCT CACAAAGCAT
CCCGTGTTCA CTCATGAAAA GTTATTTTGA AACGAGCAGC GAGTGCTCCA AGCCAGGTGT
CATATTCCTC ACCAAGAAGG GGCGGCAAGT CTGTGCCAAA CCCAGTGGTC CGGGAGTTCA
GGATTGCATG AAAAAGCTGA AGCCCTACTC AATATAATAA TAAAGAGACA AAAGAGGCCA
GCCACCCACC TCCAACACCT CCTGAGCCTC TGAAGCTCCC ACCAGGCCAG CTCTCCTCCC
ACAACAGCTT CCCACAGCAT GAAGATCTCC GTGGCTGCCA TTCCCTTCTT CCTCCTCATC
ACCATCGCCC TAGGGACCAA GACTGAATCC TCCTCACAAA CTGGGGGGAA ACCGAAGGTT
GTTAAAATAC AGCTAAAGTT GGTGGGGGGA CCTTACCACC CCTCAGAGTG CTGCTTCACC
TACACTACCT ACAAGATCCC GCGTCAGCGG ATTATGGATT ACTATGAGAC CAACAGCCAG
TGCTCCAAGC CCGGAATTGT CTTCATCACC AAAAGGGGCC ATTCCGTCTG TACCAACCCC
AGTGACAAGT GGGTCCAGGA CTATATCAAG GACATGAAGG AGAACTGAGT GACCCAGAAG
GGGTGGCGAA GGCACAGCTC AGAGACATAA AGAGAAGATG CCAAGGCCCC CTCCTCCACC
CACCGCTAAC TCTCAGCCCC AGTCACCCTC TTGGAGCTTC CCTGCTTTGA ATTAAAGACC
ACTCATGCTC TTC
kodierend für die humanen Cytokine CC-2 und CC-3 mit den nachfolgenden Aminosäuresequenzen:

CC-2:
M K
ATG AAG
V S V A A L S C L M L V A V L G S Q A Q
GTC TCC GTG GCT GCC CTC TCC TGC CTC ATG CTT GTT GCT GTC CTT GGA TCC CAG GCC CAG
F T N D A E T E L M M S K L P L E N P V
TTC ACA AAT GAT GCA GAG ACA GAG TTA ATG ATG TCA AAG CTT CCA CTG GAA AAT CCA GTA
V L N S F H F A A D C C T S Y I S Q S I
GTT CTG AAC AGC TTT CAC TTT GCT GCT GAC TGC TGC ACC TCC TAC ATC TCA CAA AGC ATC
P C S L M K S Y F E T S S E C S K P G V
CCG TGT TCA CTC ATG AAA AGT TAT TTT GAA ACG AGC AGC GAG TGC TCC AAG CCA GGT GTC
I F L T K K G R Q V C A K P S G P G V Q
ATA TTC CTC ACC AAG AAG GGG CGG CAA GTC TGT GCC AAA CCC AGT GGT CCG GGA GTT CAG
D C M K K L K P Y S I TER TER TER
GAT TGC ATG AAA AAG CTG AAG CCC TAC TCA ATA TAA TAA TAAAGAGACAAAAGAGGCCAGCCACCC
M K
ACCTCCAACACCTCCTGAGCCTCTGAAGCTCCCACCAGGCCAGCTCTCCTCCCACAACAGCTTCCCACAGC ATG AAG
I S V A A I P F F L L I T I A L G T K T
ATC TCC GTG GCT GCC ATT CCC TTC TTC CTC CTC ATC ACC ATC GCC CTA GGG ACC AAG ACT
E S S S R G P Y H P S E C C F T Y T T Y
GAA TCC TCC TCA CGG GGA CCT TAC CAC CCC TCA GAG TGC TGC TTC ACC TAC ACT ACC TAC
K I P R Q R I M D Y Y E T N S Q C S K P
AAG ATC CCG CGT CAG CGG ATT ATG GAT TAC TAT GAG ACC AAC AGC CAG TGC TCC AAG CCC
G I V F I T K R G H S V C T N P S D K W
GGA ATT GTC TTC ATC ACC AAA AGG GGC CAT TCC GTC TGT ACC AAC CCC AGT GAC AAG TGG
V Q D Y I K D M K E N
GTC CAG GAC TAT ATC AAG GAC ATG AAG GAG AAC

M K
CC-3: ATG AAG

V S V A A L S C L M L V A V L G S Q A Q
GTC TCC GTG GCT GCC CTC TCC TGC CTC ATG CTT GTT GCT GTC CTT GGA TCC CAG GCC CAG

F T N D A E T E L M M S K L P L E N P V
TTC ACA AAT GAT GCA GAG ACA GAG TTA ATG ATG TCA AAG CTT CCA CTG GAA AAT CCA GTA

V L N S F H F A A D C C T S Y I S Q S I
GTT CTG AAC AGC TTT CAC TTT GCT GCT GAC TGC TGC ACC TCC TAC ATC TCA CAA AGC ATC

P C S L M K S Y F E T S S E C S K P G V
CCG TGT TCA CTC ATG AAA AGT TAT TTT GAA ACG AGC AGC GAG TGC TCC AAG CCA GGT GTC

I F L T K K G R Q V C A K P S G P G V Q
ATA TTC CTC ACC AAG AAG GGG CGG CAA GTC TGT GCC AAA CCC AGT GGT CCG GGA GTT CAG

D C M K K L K P Y S I TER TER TER
GAT TGC ATG AAA AAG CTG AAG CCC TAC TCA ATA TAA TAA TAAAGAGACAAAAGAGGCCAGCCACCC

M K
ACCTCCAACACCTCCTGAGCCTCTGAAGCTCCCACCAGGCCAGCTCTCCTCCCACAACAGCTTCCCACAGC ATG AAG

I S V A A ! P F F L L I T I A L G T K T
ATC TCC GTG GCT GCC ATT CCC TTC TTC CTC CTC ATC ACC ATC GCC CTA GGG ACC AAG ACT

E S S S Q T G G K P K V V K I Q L K L V
GAA TCC TCC TCA CAA ACT GGG GGG AAA CCG AAG GTT GTT AAA ATA CAG CTA AAG TTG GTG

G G P Y H P S E C C F T Y T T Y K I P R
GGG GGA CCT TAC CAC CCC TCA GAG TGC TGC TTC ACC TAC ACT ACC TAC AAG ATC CCG CGT

Q R I M D Y Y E T N S Q C S K P G I V F
CAG CGG ATT ATG GAT TAC TAT GAG ACC AAC AGC CAG TGC TCC AAG CCC GGA ATT GTC TTC

I T K R G H S V C T N P S D K W V Q D Y
ATC ACC AAA AGG GGC CAT TCC GTC TGT ACC AAC CCC AGT GAC AAG TGG GTC CAG GAC TAT

I K D M K E N
ATC AAG GAC ATG AAG GAG AAC

2. Cytokin CC-2 mit der in Anspruch 1 angegebenen Aminosäuresequenz sowie dessen biologisch aktive amidierte, acetylierte, phosphorylierte und/oder glycosylierte Derivate.

3. Cytokin CC-3 mit der in Anspruch 1 angegebenen Aminosäuresequenz sowie dessen biologisch aktive amidierte, acetylierte, phosphorylierte und/oder glycosylierte Derivate.

4. Arzneimittel enthaltend Cytokin CC-2 und/oder CC-3 gemäß Anspruch 2 und/oder 3 als wirksamen Bestandteil.

5. Diagnostikmittel enthaltend poly- oder monoklonale Antikörper gegen Cytokin CC-2 und/oder 3 gemäß Anspruch 2 und/oder 3 oder für die Cytokine kodierende Nukleinsäuren, mRNA, sowie die Nukleinsäure gemäß Anspruch 1.

6. Verwendung von Cytokin CC-2 und/ oder CC-3 gemäß Ansprüchen 2 und/oder 3 zur Herstellung eines Arzneimittels zur Behandlung von Störungen der Migration von Zellen, Erkrankungen des Immunsystems, Tumoren sowie Disfunktion regulatorischer Wachstumsfunktionen.

## Claims

1. A nucleic acid of a tandem gene having the following sequences: 
Sequence 925 BP; 240 A; 296 C; 199 G; 190 T;
CCAGGAAGCA GTGAGCCCAG GAGTCCTCGG CCAGCCCTGC CTGCCCACCA GGAGGATGAA
GGTCTCCGTG GCTGCCCTCT CCTGCCTCAT GCTTGTTGCT GTCCTTGGAT CCCAGGCCCA
GTTCACAAAT GATGCAGAGA CAGAGTTAAT GATGTCAAAG CTTCCACTGG AAAATCCAGT
AGTTCTGAAC AGCTTTCACT TTGCTGCTGA CTGCTGCACC TCCTACATCT CACAAAGCAT
CCCGTGTTCA CTCATGAAAA GTTATTTTGA AACGAGCAGC GAGTGCTCCA AGCCAGGTGT
CATATTCCTC ACCAAGAAGG GGCGGCAAGT CTGTGCCAAA CCCAGTGGTC CGGGAGTTCA
GGATTGCATG AAAAAGCTGA AGCCCTACTC AATATAATAA TAAAGAGACA AAAGAGGCCA
GCCACCCACC TCCAACACCT CCTGAGCCTC TGAAGCTCCC ACCAGGCCAG CTCTCCTCCC
ACAACAGCTT CCCACAGCAT GAAGATCTCC GTGGCTGCCA TTCCCTTCTT CCTCCTCATC
ACCATCGCCC TAGGGACCAA GACTGAATCC TCCTCACGGG GACCTTACCA CCCCTCAGAG
TGCTGCTTCA CCTACACTAC CTACAAGATC CCGCGTCAGC GGATTATGGA TTACTATGAG
ACCAACAGCC AGTGCTCCAA GCCCGGAATT GTCTTCATCA CCAAAAGGGG CCATTCCGTC
TGTACCAACC CCAGTGACAA GTGGGTCCAG GACTATATCA AGGACATGAA GGAGAACTGA
GTGACCCAGA AGGGGTGGCG AAGGCACAGC TCAGAGACAT AAAGAGAAGA TGCCAAGGCC
CCCTCCTCCA CCCACCGCTA ACTCTCAGCC CCAGTCACCC TCTTGGAGCT TCCCTGCTTT
GAATTAAAGA CCACTCATGC TCTTC
and 
Sequence 973 BP; 257 A; 301 C; 215 G; 200 T;
CCAGGAAGCA GTGAGCCCAG GAGTCCTCGG CCAGCCCTGC CTGCCCACCA GGAGGATGAA
GGTCTCCGTG GCTGCCCTCT CCTGCCTCAT GCTTGTTGCT GTCCTTGGAT CCCAGGCCCA
GTTCACAAAT GATGCAGAGA CAGAGTTAAT GATGTCAAAG CTTCCACTGG AAAATCCAGT
AGTTCTGAAC AGCTTTCACT TTGCTGCTGA CTGCTGCACC TCCTACATCT CACAAAGCAT
CCCGTGTTCA CTCATGAAAA GTTATTTTGA AACGAGCAGC GAGTGCTCCA AGCCAGGTGT
CATATTCCTC ACCAAGAAGG GGCGGCAAGT CTGTGCCAAA CCCAGTGGTC CGGGAGTTCA
GGATTGCATG AAAAAGCTGA AGCCCTACTC AATATAATAA TAAAGAGACA AAAGAGGCCA
GCCACCCACC TCCAACACCT CCTGAGCCTC TGAAGCTCCC ACCAGGCCAG CTCTCCTCCC
ACAACAGCTT CCCACAGCAT GAAGATCTCC GTGGCTGCCA TTCCCTTCTT CCTCCTCATC
ACCATCGCCC TAGGGACCAA GACTGAATCC TCCTCACAAA CTGGGGGGAA ACCGAAGGTT
GTTAAAATAC AGCTAAAGTT GGTGGGGGGA CCTTACCACC CCTCAGAGTG CTGCTTCACC
TACACTACCT ACAAGATCCC GCGTCAGCGG ATTATGGATT ACTATGAGAC CAACAGCCAG
TGCTCCAAGC CCGGAATTGT CTTCATCACC AAAAGGGGCC ATTCCGTCTG TACCAACCCC
AGTGACAAGT GGGTCCAGGA CTATATCAAG GACATGAAGG AGAACTGAGT GACCCAGAAG
GGGTGGCGAA GGCACAGCTC AGAGACATAA AGAGAAGATG CCAAGGCCCC CTCCTCCACC
CACCGCTAAC TCTCAGCCCC AGTCACCCTC TTGGAGCTTC CCTGCTTTGA ATTAAAGACC
ACTCATGCTC TTC
coding for the human cytokines CC-2 and CC-3 having the following amino acid sequences:

CC-2:
M K
ATG AAG

V S V A A L S C L M L V A V L G S Q A Q
GTC TCC GTG GCT GCC CTC TCC TGC CTC ATG CTT GTT GCT GTC CTT GGA TCC CAG GCC CAG

F T N D A E T E L M M S K L P L E N P V
TTC ACA AAT GAT GCA GAG ACA GAG TTA ATG ATG TCA AAG CTT CCA CTG GAA AAT CCA GTA

V L N S F H F A A D C C T S Y I S Q S I
GTT CTG AAC AGC TTT CAC TTT GCT GCT GAC TGC TGC ACC TCC TAC ATC TCA CAA AGC ATC

P C S L M K S Y F E T S S E C S K P G V
CCG TGT TCA CTC ATG AAA AGT TAT TTT GAA ACG AGC AGC GAG TGC TCC AAG CCA GGT GTC

I F L T K K G R Q V C A K P S G P G V Q
ATA TTC CTC ACC AAG AAG GGG CGG CAA GTC TGT GCC AAA CCC AGT GGT CCG GGA GTT CAG

D C M K K L K P Y S I TER TER TER
GAT TGC ATG AAA AAG CTG AAG CCC TAC TCA ATA TAA TAA TAAAGAGACAAAAGAGGCCAGCCACCC

M K
ACCTCCAACACCTCCTGAGCCTCTGAAGCTCCCACCAGGCCAGCTCTCCTCCCACAACAGCTTCCCACAGC ATG AAG

I S V A A I P F F L L I T I A L G T K T
ATC TCC GTG GCT GCC ATT CCC TTC TTC CTC CTC ATC ACC ATC GCC CTA GGG ACC AAG ACT

E S S S R G P Y H P S E C C F T Y T T Y
GAA TCC TCC TCA CGG GGA CCT TAC CAC CCC TCA GAG TGC TGC TTC ACC TAC ACT ACC TAC

K I P R Q R I M D Y Y E T N S Q C S K P
AAG ATC CCG CGT CAG CGG ATT ATG GAT TAC TAT GAG ACC AAC AGC CAG TGC TCC AAG CCC

G I V F I T K R G H S V C T N P S D K W
GGA ATT GTC TTC ATC ACC AAA AGG GGC CAT TCC GTC TGT ACC AAC CCC AGT GAC AAG TGG

V Q D Y I K D M K E N
GTC CAG GAC TAT ATC AAG GAC ATG AAG GAG AAC

CC-3:
M K
ATG AAG
V S V A A L S C L M L V A V L G S Q A Q
GTC TCC GTG GCT GCC CTC TCC TGC CTC ATG CTT GTT GCT GTC CTT GGA TCC CAG GCC CAG
F T N D A E T E L M M S K L P L E N P V
TTC ACA AAT GAT GCA GAG ACA GAG TTA ATG ATG TCA AAG CTT CCA CTG GAA AAT CCA GTA
V L N S F H F A A D C C T S Y I S Q S I
GTT CTG AAC AGC TTT CAC TTT GCT GCT GAC TGC TGC ACC TCC TAC ATC TCA CAA AGC ATC
P C S L M K S Y F E T S S E C S K P G V
CCG TGT TCA CTC ATG AAA AGT TAT TTT GAA ACG AGC AGC GAG TGC TCC AAG CCA GGT GTC
I F L T K K G R Q V C A K P S G P G V Q
ATA TTC CTC ACC AAG AAG GGG CGG CAA GTC TGT GCC AAA CCC AGT GGT CCG GGA GTT CAG
D C M K K L K P Y S I TER TER TER
GAT TGC ATG AAA AAG CTG AAG CCC TAC TCA ATA TAA TAA TAAAGAGACAAAAGAGGCCAGCCACCC
M K
ACCTCCAACACCTCCTGAGCCTCTGAAGCTCCCACCAGGCCAGCTCTCCTCCCACAACAGCTTCCCACAGC ATG AAG
I S V A A I P F F L L I T I A L G T K T
ATC TCC GTG GCT GCC ATT CCC TTC TTC CTC CTC ATC ACC ATC GCC CTA GGG ACC AAG ACT
E S S S Q T G G K P K V V K I Q L K L V
GAA TCC TCC TCA CAA ACT GGG GGG AAA CCG AAG GTT GTT AAA ATA CAG CTA AAG TTG GTG
G G P Y H P S E C C F T Y T T Y K I P R
GGG GGA CCT TAC CAC CCC TCA GAG TGC TGC TTC ACC TAC ACT ACG TAC AAG ATC CCG CGT
Q R I M D Y Y E T N S Q C S K P G I V F
CAG CGG ATT ATG GAT TAC TAT GAG ACC AAC AGC CAG TGC TCC AAG CCC GGA ATT GTC TTC
I T K R G H S V C T N P S D K W V Q D Y
ATC ACC AAA AGG GGC CAT TCC GTC TGT ACC AAC CCC AGT GAC AAG TGG GTC CAG GAC TAT
I K D M K E N
ATC AAG GAC ATG AAG GAG AAC

2. A cytokine, CC-2, having the amino acid sequence as shown in claim 1 as well as its biologically active amidated, acetylated, phosphorylated and/or glycosylated derivatives.

3. A cytokine, CC-3, having the amino acid sequence as shown in claim 1 as well as its biologically active amidated, acetylated, phosphorylated and/or glycosylated derivatives.

4. A medicament containing cytokine CC-2 and/or CC-3 according to claim 2 and/or 3 as the active ingredient.

5. A diagnostic agent containing polyclonal or monoclonal antibodies against cytokine CC-2 and/or 3 according to claim 2 and/or 3, or nucleic acids coding for the cytokines, mRNA, as well as the nucleic acid according to claim 1.

6. Use of cytokine CC-2 and/or CC-3 according to claim 2 and/or 3 for the preparation of a medicament for the treatment of disorders of cell migration, diseases of the immune system, tumors and dysfunction of regulatory growth functions.

## Revendications

1. Acide nucléique d'un gène en tandem ayant les séquences suivantes 
Sequence 925 BP; 240 A; 296 C; 199 G; 190 T;
CCAGGAAGCA GTGAGCCCAG GAGTCCTCGG CCAGCCCTGC CTGCCCACCA GGAGGATGAA
GGTCTCCGTG GCTGCCCTCT CCTGCCTCAT GCTTGTTGCT GTCCTTGGAT CCCAGGCCCA
GTTCACAAAT GATGCAGAGA CAGAGTTAAT GATGTCAAAG CTTCCACTGG AAAATCCAGT
AGTTCTGAAC AGCTTTCACT TTGCTGCTGA CTGCTGCACC TCCTACATCT CACAAAGCAT
CCCGTGTTCA CTCATGAAAA GTTATTTTGA AACGAGCAGC GAGTGCTCCA AGCCAGGTGT
CATATTCCTC ACCAAGAAGG GGCGGCAAGT CTGTGCCAAA CCCAGTGGTC CGGGAGTTCA
GGATTGCATG AAAAAGCTGA AGCCCTACTC AATATAATAA TAAAGAGACA AAAGAGGCCA
GCCACCCACC TCCAACACCT CCTGAGCCTC TGAAGCTCCC ACCAGGCCAG CTCTCCTCCC
ACAACAGCTT CCCACAGCAT GAAGATCTCC GTGGCTGCCA TTCCCTTCTT CCTCCTCATC
ACCATCGCCC TAGGGACCAA GACTGAATCC TCCTCACGGG GACCTTACCA CCCCTCAGAG
TGCTGCTTCA CCTACACTAC CTACAAGATC CCGCGTCAGC GGATTATGGA TTACTATGAG
ACCAACAGCC AGTGCTCCAA GCCCGGAATT GTCTTCATCA CCAAAAGGGG CCATTCCGTC
TGTACCAACC CCAGTGACAA GTGGGTCCAG GACTATATCA AGGACATGAA GGAGAACTGA
GTGACCCAGA AGGGGTGGCG AAGGCACAGC TCAGAGACAT AAAGAGAAGA TGCCAAGGCC
CCCTCCTCCA CCCACCGCTA ACTCTCAGCC CCAGTCACCC TCTTGGAGCT TCCCTGCTTT
GAATTAAAGA CCACTCATGC TCTTC
et 
Sequence 973 BP; 257 A; 301 C; 215 G; 200 T;
CCAGGAAGCA GTGAGCCCAG GAGTCCTCGG CCAGCCCTGC CTGCCCACCA GGAGGATGAA
GGTCTCCGTG GCTGCCCTCT CCTGCCTCAT GCTTGTTGCT GTCCTTGGAT CCCAGGCCCA
GTTCACAAAT GATGCAGAGA CAGAGTTAAT GATGTCAAAG CTTCCACTGG AAAATCCAGT
AGTTCTGAAC AGCTTTCACT TTGCTGCTGA CTGCTGCACC TCCTACATCT CACAAAGCAT
CCCGTGTTCA CTCATGAAAA GTTATTTTGA AACGAGCAGC GAGTGCTCCA AGCCAGGTGT
CATATTCCTC ACCAAGAAGG GGCGGCAAGT CTGTGCCAAA CCCAGTGGTC CGGGAGTTCA
GGATTGCATG AAAAAGCTGA AGCCCTACTC AATATAATAA TAAAGAGACA AAAGAGGCCA
GCCACCCACC TCCAACACCT CCTGAGCCTC TGAAGCTCCC ACCAGGCCAG CTCTCCTCCC
ACAACAGCTT CCCACAGCAT GAAGATCTCC GTGGCTGCCA TTCCCTTCTT CCTCCTCATC
ACCATCGCCC TAGGGACCAA GACTGAATCC TCCTCACAAA CTGGGGGGAA ACCGAAGGTT
GTTAAAATAC AGCTAAAGTT GGTGGGGGGA CCTTACCACC CCTCAGAGTG CTGCTTCACC
TACACTACCT ACAAGATCCC GCGTCAGCGG ATTATGGATT ACTATGAGAC CAACAGCCAG
TGCTCCAAGC CCGGAATTGT CTTCATCACC AAAAGGGGCC ATTCCGTCTG TACCAACCCC
AGTGACAAGT GGGTCCAGGA CTATATCAAG GACATGAAGG AGAACTGAGT GACCCAGAAG
GGGTGGCGAA GGCACAGCTC AGAGACATAA AGAGAAGATG CCAAGGCCCC CTCCTCCACC
CACCGCTAAC TCTCAGCCCC AGTCACCCTC TTGGAGCTTC CCTGCTTTGA ATTAAAGACC
ACTCATGCTC TTC
codant pour les cytokines humaines CC-2 et CC-3 ayant les séquences d'acides aminés suivantes :

CC-2:
M K
ATG AAG

V S V A A L S C L M L V A V L G S Q A Q
GTC TCC GTG GCT GCC CTC TCC TGC CTC ATG CTT GTT GCT GTC CTT GGA TCC CAG GCC CAG

F T N D A E T E L M M S K L P L E N P V
TTC ACA AAT GAT GCA GAG ACA GAG TTA ATG ATG TCA AAG CTT CCA CTG GAA AAT CCA GTA

V L N S F H F A A D C C T S Y I S Q S I
GTT CTG AAC AGC TTT CAC TTT GCT GCT GAC TGC TGC ACC TCC TAC ATC TCA CAA AGC ATC

P C S L M K S Y F E T S S E C S K P G V
CCG TGT TCA CTC ATG AAA AGT TAT TTT GAA ACG AGC AGC GAG TGC TCC AAG CCA GGT GTC

I F L T K K G R Q V C A K P S G P G V Q
ATA TTC CTC ACC AAG AAG GGG CGG CAA GTC TGT GCC AAA CCC AGT GGT CCG GGA GTT CAG

D C M K K L K P Y S I TER TER TER
GAT TGC ATG AAA AAG CTG AAG CCC TAC TCA ATA TAA TAA TAAAGAGACAAAAGAGGCCAGCCACCC

M K
ACCTCCAACACCTCCTGAGCCTCTGAAGCTCCCACCAGGCCAGCTCTCCTCCCACAACAGCTTCCCACAGC ATG AAG

I S V A A I P F F L L I T I A L G T K T
ATC TCC GTG GCT GCC ATT CCC TTC TTC CTC CTC ATC ACC ATC GCC CTA GGG ACC AAG ACT

E S S S R G P Y H P S E C C F T Y T T Y
GAA TCC TCC TCA CGG GGA CCT TAC CAC CCC TCA GAG TGC TGC TTC ACC TAC ACT ACC TAC

K I P R Q R I M D Y Y E T N S Q C S K P
AAG ATC CCG CGT CAG CGG ATT ATG GAT TAC TAT GAG ACC AAC AGC CAG TGC TCC AAG CCC

G I V F I T K R G H S V C T N P S D K W
GGA ATT GTC TTC ATC ACC AAA AGG GGC CAT TCC GTC TGT ACC AAC CCC AGT GAC AAG TGG

V Q D Y I K D M K E N
GTC CAG GAC TAT ATC AAG GAC ATG AAG GAG AAC

CC-3:
M K
ATG AAG
V S V A A L S C L M L V A V L G S Q A Q
GTC TCC GTG GCT GCC CTC TCC TGC CTC ATG CTT GTT GCT GTC CTT GGA TCC CAG GCC CAG
F T N D A E T E L M M S K L P L E N P V
TTC ACA AAT GAT GCA GAG ACA GAG TTA ATG ATG TCA AAG CTT CCA CTG GAA AAT CCA GTA
V L N S F H F A A D C C T S Y I S Q S I
GTT CTG AAC AGC TTT CAC TTT GCT GCT GAC TGC TGC ACC TCC TAC ATC TCA CAA AGC ATC
P C S L M K S Y F E T S S E C S K P G V
CCG TGT TCA CTC ATG AAA AGT TAT TTT GAA ACG AGC AGC GAG TGC TCC AAG CCA GGT GTC
I F L T K K G R Q V C A K P S G P G V Q
ATA TTC CTC ACC AAG AAG GGG CGG CAA GTC TGT GCC AAA CCC AGT GGT CCG GGA GTT CAG
D C M K K L K P Y S I TER TER TER
GAT TGC ATG AAA AAG CTG AAG CCC TAC TCA ATA TAA TAA TAAAGAGACAAAAGAGGCCAGCCACCC
M K
ACCTCCAACACCTCCTGAGCCTCTGAAGCTCCCACCAGGCCAGCTCTCCTCCCACAACAGCTTCCCACAGC ATG AAG
I S V A A I P F F L L I T I A L G T K T
ATC TCC GTG GCT GCC ATT CCC TTC TTC CTC CTC ATC ACC ATC GCC CTA GGG ACC AAG ACT
E S S S Q T G G K P K V V K I Q L K L V
GAA TCC TCC TCA CAA ACT GGG GGG AAA CCG AAG GTT GTT AAA ATA CAG CTA AAG TTG GTG
G G P Y H P S E C C F T Y T T Y K I P R
GGG GGA CCT TAC CAC CCC TCA GAG TGC TGC TTC ACC TAC ACT ACG TAC AAG ATC CCG CGT
Q R I M D Y Y E T N S Q C S K P G I V F
CAG CGG ATT ATG GAT TAC TAT GAG ACC AAC AGC CAG TGC TCC AAG CCC GGA ATT GTC TTC
I T K R G H S V C T N P S D K W V Q D Y
ATC ACC AAA AGG GGC CAT TCC GTC TGT ACC AAC CCC AGT GAC AAG TGG GTC CAG GAC TAT
I K D M K E N
ATC AAG GAC ATG AAG GAG AAC

2. Cytokine CC-2 ayant la séquence d'acides aminés indiquée dans la revendication 1 ainsi que ses dérivés biologiquement actifs amidés, acétylés, phosphorylés et/ou glycosylés.

3. Cytokine CC-3 ayant la séquence d'acides aminés indiquée dans la revendication 1 ainsi que ses dérivés biologiquement actifs amidés, acétylés, phosphorylés et/ou glycosylés.

4. Médicament contenant de la cytokine CC-2 et/ou CC-3 selon la revendication 2 et/ou 3 en tant que constituant actif.

5. Agent de diagnostic contenant des anticorps poly- ou monoclonaux contre la cytokine CC-2 ou CC-3 selon la revendication 2 et/ou 3, ou des acides nucléiques codant pour les cytokines, des ARNm ainsi que l'acide nucléique selon la revendication 1.

6. Utilisation de cytokine CC-2 et/ou de cytokine CC-3 selon les revendications 2 et/ou 3 pour préparer un médicament destiné au traitement des troubles de la migration des cellules, des maladies du système immunitaire, des tumeurs ainsi que du dysfonctionnement des fonctions régulatrices de croissance.
